# EUROPEAN PATENT SPECIFICATION

(11) **EP 3 680 912 B1**
(45) Date of publication and mention of the grant of the patent: **29.06.2022**
(21) Application number: 19151150.0
(22) Date of filing: 10.01.2019
(51) Int. Cl.: G16H 30/40, G16H 50/20

(54) **TECHNIQUE FOR PERFORMING A QUALITY ASSESSMENT FOR A MEDICAL IMAGE**
VERFAHREN ZUR DURCHFÜHRUNG EINER QUALITÄTSBEURTEILUNG FÜR EIN MEDIZINISCHES BILD
TECHNIQUE POUR EFFECTUER UNE ÉVALUATION DE LA QUALITÉ D'UNE IMAGE MÉDICALE

(43) Date of publication of application: 15.07.2020
(73) Proprietor: medneo GmbH, 10117 Berlin (DE)
(72) Inventor: HARTKENS, Thomas, 13086 Berlin (DE); ISSING, Matthias, 12307 Berlin (DE); MEZEY, David, 13357 Berlin (DE); VOGT, Johannes, 10827 Berlin (DE); JUNEJA, Medha, 10781 Berlin (DE)
(74) Representative: Laqua, Bernd Christian Kurt

(56) References cited:
- EP-A1- 3 392 832
- EP-A2- 1 881 453
- JP-A- 2009 207 585
- US-A1- 2004 193 053
- US-A1- 2011 301 447
- US-A1- 2016 364 857
- US-A1- 2018 137 244

## Description

### Technical Field

The present disclosure generally relates to the field of medical imaging systems. In particular, a method and system for performing a feedback-based quality assessment for a medical image acquired by a medical imaging device is presented.

### Background

Quality control of medical images, such as magnetic resonance (MR) or computer tomography (CT) images, has been an intense field of research in the last decades. Generally, it is important to exclude problematic image acquisitions and avoid bias in the subsequent reading of images by physicians. Since visual inspection to detect images of bad quality is not always practical in the daily routine of radiological centers, quantitative image quality measurement techniques have been introduced which allow automatically verifying image quality after acquisition. However, quantitative measurements may not capture all aspects of image quality and, thus, quality-controlled acquisition based on purely quantitative measurements may not differentiate images sufficiently.

Image quality can be influenced by many variables, such as the acquisition parameters of the imaging device, the patient positioning in the medical imaging device (e.g., a scanner), the training of the staff, and the standard operating procedures (SOPs) to be applied for image acquisitions. In particular, an imaging device typically holds several hundreds or even more than a thousand different acquisition parameter protocols, which may be set depending on the specific needs of an examination. Radiological centers continuously modify those variables aiming to enhance the resulting image quality. However, especially in case of geographically distributed imaging devices (e.g., located in different buildings or different cities), the control of image quality may become challenging because all sites must generally use the same and up-to-date variables to achieve comparable image quality across all locations and to meet the expectations of the radiologists.

In case of distributed radiological centers, the following problems may particularly arise when it comes to the control of image quality. In conventional systems, on-site technicians must manually select the appropriate acquisition parameters from a list of generic acquisition protocols locally installed on the medical imaging device (also called "modality"). Due to the manual selection, a technician may choose - either by accident or by lack of experience - a wrong acquisition protocol for a particular patient. As the acquisition protocols are stored locally at each individual imaging device, inconsistencies in the parameters may occur across sites and the resulting imaging quality may hence vary between different radiological centers. If the acquisition parameters of an acquisition protocol stored at one site are not up-to-date, an obsolete protocol may be applied by the technician and, if SOPs are not properly synchronized with the selected acquisition parameters, motion artifacts may be introduced or wrong fields of view may be acquired, e.g., when the patient is not properly positioned in the scanner in accordance with an SOP. For a reliable diagnosis, the radiologist (e.g., working from a remote location) may need adapted acquisition parameters for a specific patient. As technicians typically adjust those parameters by themselves or after verbal communication with the radiologist, inappropriate adjustments may occur. Also, technicians at the modality may not recognize problems with the image quality and automatic quantitative quality measurements may not determine whether sufficient image quality is given with satisfying certainty, as described above. As a result, images of inferior quality may be sent to the radiologists and erroneous conclusions by the radiologists may be the consequence. Also, a re-acquisition of the image may not be possible since the patient may have left the imaging device by the time the radiologist reviews an image of inferior quality.

Document EP 3 392 832 A1 discloses an image data processing system including an anatomy detector to detect an anatomy in an image and to remove items not included in the anatomy from the image. The system includes a bounding box generator to generate a bounding box around a region of interest in the anatomy. The system includes a voxel-level segmenter to classify image data within the bounding box at the voxel level to identify an object in the region of interest. The system includes an output imager to output an indication of the object identified in the region of interest segmented in the image.

Document US 2018/0137244 A1 discloses an artificial intelligence findings system. An image identification engine receives new image data pertaining to a patient and past reports and image data pertaining to the patient. The image identification engine uses the past image data pertaining to the patient to identify for study images of patient anatomical structures, anatomical anomalies and anatomical features within the new image data. A findings engine receives new image data and processes the new image data to generate findings based on the new image data and based on the identified for study images of patient anatomical structures, anatomical anomalies and anatomical features within the new image data. A communication interface provides the findings to a diagnostic review system that presents the findings to a user. An adjustment engine receives notifications of changes to findings made by a user when using the medical image interpretation system.

Document US 2004/0193053 A1 discloses that, for the purpose of suitably making comparison between a previously acquired reference image and a real-time image currently being acquired, a reference image and a scan condition therefor are stored, the reference image and scan condition are read out, a real-time image is acquired after setting the scan condition, and the reference image and real-time image are displayed side by side.

Document US 2016/0364857 A1 discloses methods and systems for automatically determining image characteristics serving as a basis for a diagnosis associated with an image study type. One system includes a server including an electronic processor and an interface for communicating with at least one data source. The electronic processor is configured to receive an image study from the at least one data source over the interface. The image study being of the image study type and including a plurality of images. The electronic processor is also configured to determine an image characteristic for each of the plurality of images and determine whether each of the plurality of images was used to establish a diagnosis. The electronic processor is also configured to store the image characteristic for each of the plurality of images and an indicator of whether each of the plurality of images was used to establish the diagnosis in a data structure.

### Summary

There is a need for a technique for use in a medical imaging system that avoids one or more of the problems discussed above, or other problems.

According to the present invention, a method and a system for performing feedback-based quality assessment for a medical image according to the independent claims is provided. Developments are set forth in the dependent claims.

According to a first aspect, a method for performing a feedback-based quality assessment for a medical image acquired by a medical imaging device is provided. The method comprises determining a level of image quality for the acquired medical image using an assessment component configured to assess image quality levels based on feedback of physicians collected for previously acquired medical images.

The medical imaging device and the assessment component are part of a medical imaging system. Prior to determining the level of image quality, the medical image is acquired by the medical imaging device and, upon completion of the acquisition, the assessment of the image quality level is carried out. The assessment of the image quality level performed by the assessment component may (but does not necessarily have to) include applying quantitative image quality measurements, such as the conventional quantitative quality measurement techniques mentioned above. Rather than purely relying on quantitative image quality measurements for the determination of the image quality level of the acquired medical image, however, the assessment component carries out the assessment based on feedback of physicians (e.g., radiologists) collected for previously acquired medical images. Such assessment may include calculating predictions of satisfaction levels of physicians using artificial intelligence-based techniques like deep learning, for example. The assessment component thus comprises a machine learning module trained to predict a satisfaction level of a physician or a group of physicians based on the feedback of physicians collected for previously acquired medical images. If the machine learning module is trained to predict the satisfaction level for a group of physicians, the satisfaction level may be representative of a general image quality (e.g., independently from personal preferences of a particular physician). If the machine module is trained to predict the satisfaction level for a particular physician, on the other hand, the satisfaction level may be representative of a subjective image quality specifically taking into account the preferences of the particular physician.

The result of the image quality level assessment may be displayed by the assessment component to a user (e.g., an on-site technician) with an indication of the predicted satisfaction level, so that the user may know whether the physician in charge of the examination will prospectively be satisfied with the acquired medical image or not. When the physician will likely be satisfied (e.g., when the determined level of image quality exceeds a predetermined threshold), the acquired medical image is provided to the physician for review. This comprises transmitting the acquired medical image to a workstation of the physician (in particular, when the physician works from a remote location). On the other hand, when the physician will likely not be satisfied (e.g., when the determined level of image quality does not exceed the predetermined threshold), the acquisition of the medical image is re-run with an adjusted configuration of the medical imaging device within the same examination, i.e., without the patient having to leave the scanner. When acquiring the medical image is performed as part of an examination, the method thus further comprises repeating acquiring the medical image by the medical imaging device with a different configuration within the (same) examination.

Upon receipt of the acquired medical image for review, the physician may rate the medical image using a feedback component provided in the medical imaging system to provide feedback on the quality level of the medical image as perceived by the physician. Such feedback may systematically be collected and incorporated into the collection of feedback of physicians for previously acquired medical images mentioned above. The newly collected feedback may be used to reassess or refine a selection of a plurality of reference images (the use of reference images in accordance with the present disclosure will be described in more detail below) and/or to refine the accuracy of the machine learning module which predicts the satisfaction levels. The method thus further comprises collecting feedback on the image quality level of the acquired medical image from a physician and using the collected feedback for selecting reference images representative of desired image quality levels for particular types of medical images. The method also comprises collecting feedback on the image quality level of the acquired medical image from a physician and using the collected feedback for training the machine learning module to predict satisfaction levels of physicians or groups of physicians for medical images, as described above. A feedback loop is therefore introduced which allows a continuous quality improvement of images acquired in the medical imaging system. This may even apply across multiple sites when the medical imaging device is one of a plurality of medical imaging devices distributed across a plurality of sites (e.g., plural radiological centers in different buildings or different cities).

In order to configure the medical imaging device by a set of acquisition parameters for appropriately acquiring the medical image, the method further comprises selecting, prior to acquiring the medical image, a reference image from a plurality of reference images, wherein each of the plurality of reference images is stored in association with a set of acquisition parameters which has been used to acquire the respective reference image, and configuring the medical imaging device by the set of acquisition parameters stored in association with the selected reference image for acquiring the medical image. Rather than configuring the medical imaging device by selecting a generic acquisition protocol stored locally at the medical imaging device, as described above, the medical imaging device is thus configured by selecting a reference image. As the reference image is stored in association with the set of acquisition parameters which has been used to acquire the reference image, the medical imaging device is configured by the same configuration that was used to acquire the reference image, in the expectation that the resulting imaging quality for the acquired image may be comparable to that of the reference image. The reference image is selected from a plurality of reference images (e.g., from a pool of reference images stored in a repository) by a user, such as a physician (e.g., a radiologist) or other medical personnel. The selection of the reference image may be performed using a composition component of the medical imaging system which may be configured to receive a corresponding selection of the user (e.g., via a user interface). The composition component may be part of the medical imaging device itself or may be provided as a separate component in the medical imaging system. Once selected, the set of acquisition parameters stored in association with the selected reference image are applied by a configuration component to the medical imaging device to effectively configure the medical imaging device. If the composition component is a separate component, the selected reference image may be transferred to the medical imaging device, e.g., using the digital imaging and communications in medicine (DICOM) standard, and, once transferred, the set of acquisition parameters stored in association with the selected reference image (e.g., stored together with the reference image in a DICOM file) may be applied to the medical imaging device.

Each of the plurality of reference images may represent a sample image usable as template for future image acquisitions. Each of the plurality of reference images may previously be selected as being representative of a desired level of image quality for a particular type of medical image. In other words, the plurality of reference images may correspond to images which are selected to be of "good" quality for particular types of medical images. The particular type of a medical image may relate to a particular body region or a particular anatomical part, for example. The plurality of reference images may be selected (or "exported") from a picture archiving and communication system (PACS), which may store a collection of previously acquired medical images. The selection of the plurality of reference images from the PACS may be performed by users with medical expertise, such as physicians (e.g., radiologists) or other medical personnel, for example. In another variant, the plurality of reference images may be determined automatically based on statistical methods or machine learning techniques and may be based on feedback on the image quality levels of the reference images previously collected from physicians, for example.

The medical imaging device may be a device configured to acquire structural or functional images of a body, including modalities using MR, CT, radiography, ultrasound, nuclear medicine and visible light, for example. The medical images acquired by the medical imaging device may correspond to MR images, CT images, X-ray images, etc. accordingly. Acquisition parameters may generally be used to configure the medical imaging device for a particular acquisition to be performed. As a mere example, typical acquisition parameters applied to an MR scanner may comprise definitions of a repetition time, an echo time, a number of signal averages, a slice thickness, an acquisition plane and a field of view, for example, which correspond to acquisition parameters according to the present invention.

As said, the medical imaging device is part of a medical imaging system and may be installed at a particular site (e.g., at a radiological center). In one variant, the medical imaging device may be one of a plurality of medical imaging devices distributed across a plurality of sites (e.g., plural radiological centers in different buildings or different cities), wherein the plurality of reference images may be stored in a central repository configured to distribute the plurality of reference images to the plurality of medical imaging devices. In the central repository, the plurality of reference images may be centrally version controlled. In each of the plurality of sites, a local subset of the central repository may be maintained, wherein each local subset (or "local repository instance") may be synchronized with the central repository. The required data transfer may be realized over corresponding network connections, such as over the Internet, for example. A local repository instance may form the basis from which the reference image is selected by means of the composition component, as described above.

In the central repository, the plurality of reference images may be categorized by at least one of medical imaging device types, standard image sets and/or physician specific image sets, body regions, and anatomical parts. The central repository may thus be organized in categories to provide support for different types of imaging devices and to facilitate the retrieval of reference images. Among the above-mentioned categories, the category "medical imaging device type" may indicate for a reference image a type of medical imaging device on which the reference image has been acquired, the category "standard image set" may indicate for a reference image that the reference image is representative of a generally recommended set of acquisition parameters (e.g., independently from personal preferences of a particular physician), the category "physician specific image set" may indicate for a reference image that the reference image is representative of a set of acquisition parameters preferred by a particular physician, the category "body region" may indicate for a reference image a body region for which the reference image is representative (e.g., head, lower extremities, etc.), and the category "anatomical part" may indicate for a reference image an actual anatomical part for which the reference image is representative (e.g., ankle, foot, knee, etc.). Local repository instances may be organized in the same manner.

In the central repository, each of the plurality of reference images may further be stored in association with an SOP to be adhered to when acquiring the medical image using the set of acquisition parameters stored in association with the respective reference image. The SOP may contain instructions relating to variables not covered by the set of acquisition parameters stored in association with the respective reference image, such as instructions for the positioning of a patient (e.g., the positioning and orientation of a patient's body part in a scanner). While the set of acquisition parameters stored in association with the selected reference image may be applied to the medical imaging device directly, as described above, it will be understood that the set of acquisition parameters stored in association with the selected reference image may also be adjusted prior to configuring the medical imaging device. Such adjustments may be carried out using the composition component mentioned above, for example.

In order to obtain a whole series of images for a complete examination of a patient, sequences of reference images may be defined. In the central repository, sequences of images may thus be stored, wherein the medical imaging device may be configurable by each of the sequences to acquire a respective series of medical images in accordance with the sets of acquisition parameters stored in association with the reference images of the respective sequence. Instead of selecting a single reference image and configuring the medical imaging device based on the selected single reference image, as described above, the method may thus also comprise selecting a sequence of reference images from the sequences of reference images, and configuring the medical imaging device to acquire a series of medical images in accordance with the sets of acquisition parameters stored in association with the reference images of the selected sequence. In order to avoid duplicate storage of reference images in the central repository, reference images of the sequences may be stored by reference (or "be linked") to corresponding reference images of the plurality of reference images stored in the central repository.

A maintenance component configured to maintain consistency for these links may be provided. The maintenance component may be configured to update corresponding links if a reference image is removed to prevent a degenerated repository. The maintenance component may also be configured to identify reference images with similar acquisition parameters and, if reference images with sufficiently similar acquisition parameters are identified, to remove such reference images from the repository. While the selected sequence may be applied to the medical imaging device directly, as described above, it will be understood that a sequence selected from the sequences of reference images may be adjusted prior to configuring the medical imaging device with the selected sequence. For example, one or more reference images may be added or removed from the sequence in order to create a patient specific examination specifically adapted for a particular patient. Such adjustments may be carried out using the composition component mentioned above.

According to a second aspect, a system for performing a feedback-based quality assessment for a medical image acquired by a medical imaging device is provided. The system comprises an assessment component configured to determine a level of image quality for the acquired medical image, wherein the assessment component assesses image quality levels based on feedback of physicians collected for previously acquired medical images.

The system according to the second aspect corresponds to the medical imaging system described above in relation to the method of the first aspect. Therefore, all features described above in relation to the medical imaging system may be comprised by the system of the second aspect as well, including all aspects of the plurality of medical imaging devices, the central repository, the local repository instances and all components mentioned above, including the configuration component, the composition component, the maintenance component, the assessment component and the feedback component. Unnecessary repetitions are thus omitted in the following.

### Brief Description of the Drawings

In the following, the present disclosure will further be described with reference to exemplary embodiments illustrated in the figures, in which:
- Figure 1: illustrates an overview of a method which may be performed in a medical imaging system according to the present disclosure;
- Figure 2: illustrates an exemplary composition of a computing unit on which one or more of the components of a medical imaging system according to the present disclosure may be executed;
- Figure 3: illustrates an overview of the feedback loop in the acquisition process according to the present disclosure;
- Figure 4: illustrates an exemplary repository structure for use in a medical imaging system according to the present disclosure;
- Figure 5: illustrates an overview of a medical imaging system and its components according to the present disclosure; and
- Figure 6: illustrates an exemplary view indicating a predicted satisfaction level of a physician according to the present disclosure.

### Detailed Description

In the following description, for purposes of explanation and not limitation, specific details are set forth in order to provide a thorough understanding of the present disclosure. It will be apparent to one skilled in the art that the present disclosure may be practiced in other implementations that depart from these specific details. In particular, while the following description particularly relates to a system comprising radiological centers, it will be understood that the present disclosure is not limited thereto and that the technique presented herein may be practiced with any other medical imaging system.

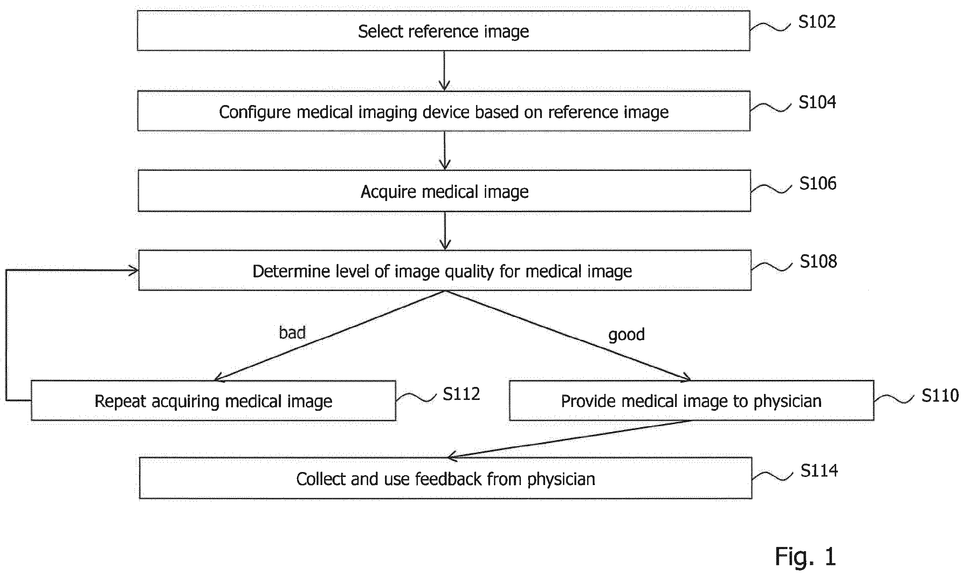

Figure 1 illustrates an overview of a method which is performed in a medical imaging system according to the present disclosure. The method is dedicated to configuring a medical imaging device by a set of acquisition parameters for acquiring a medical image and to performing a feedback-based quality assessment for the acquired medical image. The method of Figure 1 generally corresponds to the method described above. Unnecessary repetitions are thus omitted in the following.

In step S102, the method comprises selecting a reference image from a plurality of reference images, wherein each of the plurality of reference images is stored in association with a set of acquisition parameters which has been used to acquire the respective reference image. In step S104, the method comprises configuring the medical imaging device by the set of acquisition parameters stored in association with the selected reference image for acquiring the medical image. In step S106, the method comprises acquiring the medical image by the medical imaging device. In step S108, the method comprises determining a level of image quality of the acquired medical image using an image quality assessment component configured to assess image quality levels based on feedback of physicians collected for previously acquired medical images. When the determined level of image quality exceeds a predetermined threshold (i.e., is determined to be "good"), the method further comprises providing, in step S110, the acquired medical image to a physician. When the determined level of image quality does not exceed the predetermined threshold (i.e., is determined to be "bad"), the method comprises repeating, in step S112, acquiring the medical image by the medical imaging device with a different configuration within the same examination. Re-acquiring the medical image in accordance with step S112 is performed repeatedly until the determined level of image quality, as determined in step S108, is good enough to provide the acquired medical image to the physician in accordance with step S110. In step S114, the method further comprises collecting feedback on the image quality level of the acquired medical image from the physician and using the feedback for improvements of the medical imaging system, such as using the collected feedback for selecting reference images representative of the desired image quality levels for particular types of medical images and/or using the collected feedback for training a machine learning module to predict satisfaction levels of physicians or groups of physicians for medical images.

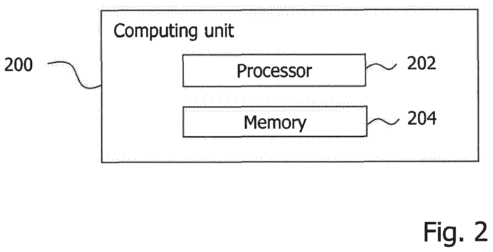

Figure 2 illustrates an exemplary composition of a computing unit 200 on which one or more of the components of the medical imaging system according to the present disclosure may be executed. Thus, each of the components of the medical imaging system described herein may be executed on a computing unit like computing unit 200. These components may in particular include the plurality of medical imaging devices, the central repository, the local repositories, the configuration component, the composition component, the maintenance component, the assessment component, and the feedback component. The computing unit 200 may comprise at least one processor 202 and at least one memory 204, wherein the at least one memory 204 may contain instructions executable by the at least one processor 202 such that the respective computing unit 200 is operable to carry out the functions of the respective component described herein.

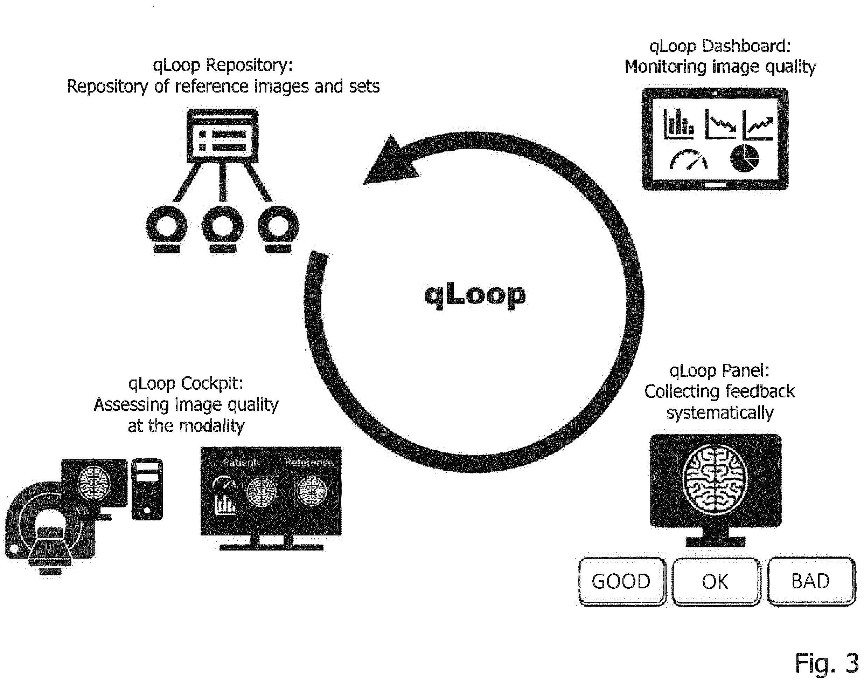

Figure 3 illustrates a general overview of the feedback loop in the acquisition process introduced by the present disclosure. The feedback loop (denoted as quality loop "qLoop" in the figure) may generally aim at measuring, controlling, assuring and/or improving the quality of medical images acquired in the medical imaging system according to the present disclosure. Acquisition parameters may be managed based on reference images instead of conventional generic acquisition protocols stored locally on the medical imaging devices. Images of "good" quality may be exported from a PACS and imported as reference images into a qLoop repository (corresponding to the "central repository" described above). Example images selected from the PACS may become templates for future image acquisitions and the acquisition process may thus generally be denoted as "scan by example". Configuring the medical imaging device by reference images may allow radiologists to define the desired acquisition parameters in an easy and intuitive manner, i.e., by just pointing to a representative sample image which has been rated to be "good". Instead of configuring a generic acquisition protocol stored locally at the medical imaging device, the radiologist may thus not need to be physically at the imaging device but may rather select the reference image for configuring the medical imaging device from a remote location.

Besides mere reference images, the repository may contain predefined collections of reference images, called "sets" (corresponding to the "sequences of reference images" described above). Such sets may combine reference images into a program, i.e., into a sequence of instructions for the imaging device to acquire a series of medical images required for a complete examination of a patient. The repository including the reference images and sets may be shared and synchronized between multiple radiological centers so that radiologists may benefit from the above-mentioned advantages in all radiological centers of the system.

For a specific examination, a reference image or set may be selected (and, optionally, adjusted, such as by adapting acquisition parameters associated with a reference image or by adding/removing reference images from a set) by the radiologist using the composition component. The reference image or the set may then be sent to the imaging device to configure the imaging device accordingly and start the acquisition. After the acquisition, the acquired image or sequence of images may be quality controlled at a qLoop cockpit (corresponding to the "assessment component" described above) by applying automatic image quality checks based on feedback of physicians collected for previously acquired medical images (and, optionally, based on conventional quantitative image quality measurements).

If the acquired image or sequence of images passes the quality check, it may be sent for review to the radiologist who may evaluate its quality as part of the usual reading and who may rate the quality at a qLoop panel (corresponding to the "feedback component" described above) by indicating a corresponding level of satisfaction (cf. "good", "ok" and "bad" in Figure 3). Such feedback of radiologists may be systematically collected, centrally monitored at a qLoop dashboard and used to identify improvement potential with respect to the selection of reference images in the repository. In the centrally-managed repository, changes reflecting such improvements may be introduced to thereby close the qLoop and provide for a continuous process of improving reference images based on feedback given by radiologists. The feedback process may generally be scalable, i.e., an unlimited number of radiologists may provide feedback in parallel for images acquired in multiple radiological centers.

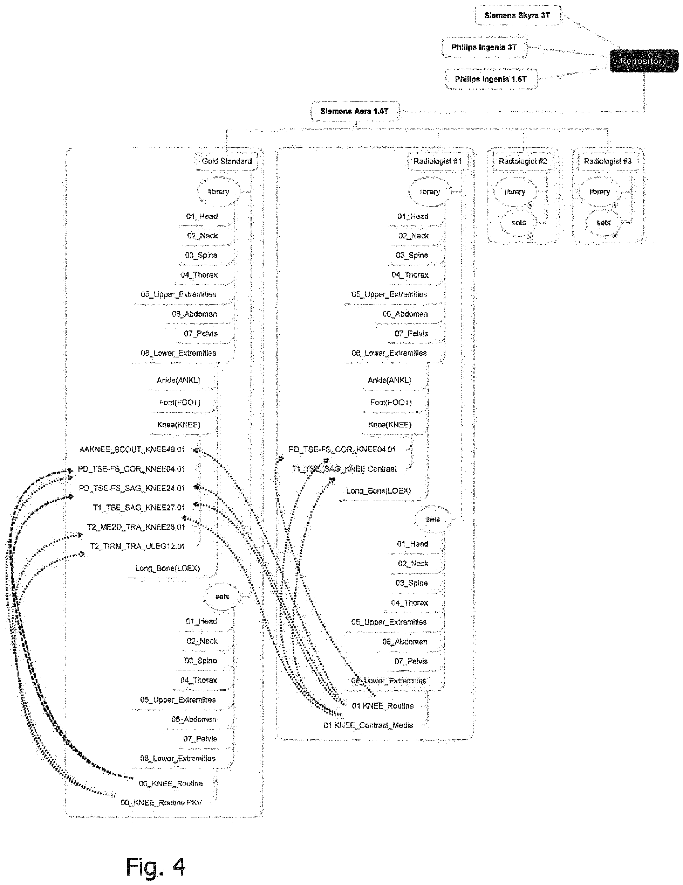

An exemplary structure of the repository is depicted in Figure 4 which shows that the repository may be organized in accordance with plural categories. In the shown example, the categories are reflected by "levels" to provide support for different imaging devices types and to facilitate the retrieval of reference images. In the example of Figure 4, the repository supports an indication of medical imaging device types at the top level (corresponding to the category "medical imaging device type" described above), like Siemens Area 1.5T, Philips Ingenia 3T, etc. At the second level, the repository supports an indication of a standard image set representative of generally recommended acquisition parameters (cf. "gold standard" in the figure, corresponding to the category "standard image set" described above) as well as indications of physician specific image sets representative of acquisition parameters preferred by a particular physician (corresponding to the category "physician specific image set" described above). Due to the feedback loop, the reference images of the standard image set may be improved over time and, if a particular radiologist has specific needs, specific reference images may be stored under the physician specific image set of that radiologist. At the third level of the repository, the actual reference images may be stored (cf. the "library" sections in the figure). At this level, the reference images may be organized with respect to body regions, e.g., head, lower extremities, etc., (corresponding to the category "body region" described above) as well as actual anatomical parts to be scanned, e.g., ankle, foot, knee, etc. (corresponding to the category "anatomical part" described above). Each reference image may be stored in association with an SOP which contains instructions regarding the required patient positioning, or the like. Sequences of reference images, i.e., sets, representing a series of acquisitions may be stored in the "sets" section at the third level. Similar to the reference images in the "library" section, the "sets" section may be organized with respect to body regions and anatomical parts and each set may be stored in association with a corresponding SOP.

The arrows in Figure 4 visualize exemplary links from sets of the "sets" section to reference images in the "library" section, illustrating that reference images of the sets may be stored by reference in order to avoid duplicate storage of reference images. For example, the set "00_KNEE_Routine" includes a link to the reference images "PD_TSE-FS_COR_KNEE04.01" and "PD_TSE-FS_SAG_KNEE24.01" included in the "library" section of the standard image set. Links may also range from a radiologist specific set (cf. "01 KNEE_Contrast_Media" in the figure) to reference images in the library of the standard image set, for example.

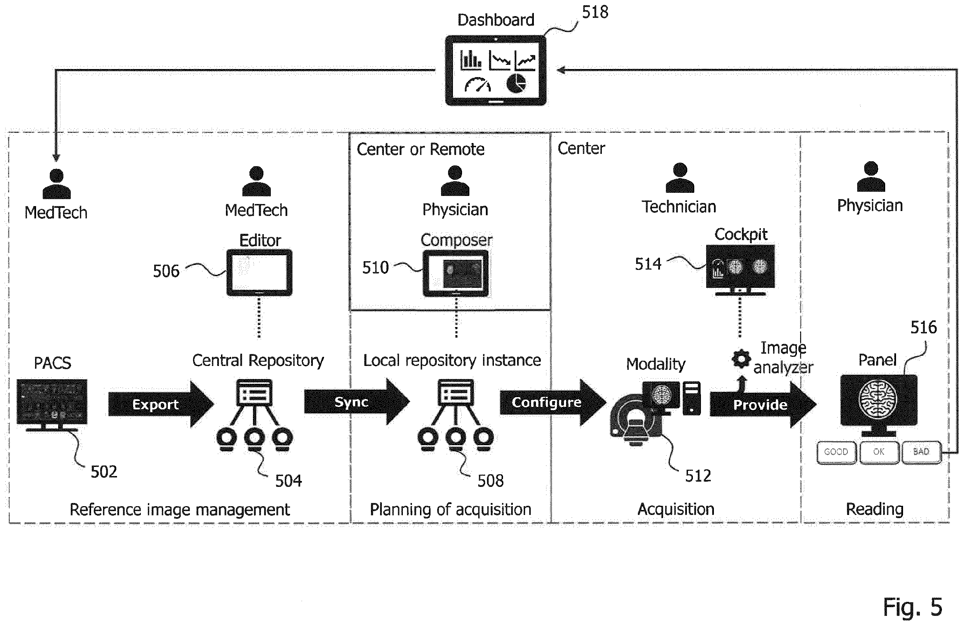

Figure 5 illustrates an overview of a medical imaging system according to the present disclosure. In the medical imaging system, a PACS 502 may serve as a source for selecting reference images. From the PACS 502, medical images of "good" quality may be exported together with their associated acquisition parameters and incorporated as reference images into the central repository 504. Using an editor component 506 (corresponding to the "maintenance component" described above), the consistency of the repository may be maintained, e.g., links to reference images may be kept up-to-date and reference images with similar acquisition parameters may be identified. If reference images with sufficiently similar acquisition parameters are found, such reference images may be removed from the repository and corresponding links may be updated to prevent a degenerated repository.

Each radiological center of the system may maintain a local repository instance 508 which may be synchronized with the central repository 504, i.e., each diagnostic center may store a local subset of the central repository 504, e.g., depending on which radiologists work in this center. The local repository instance 508 may form the basis to select a reference image or a set for a specific examination using a composer component 510 (corresponding to the "composition component" described above). The composer component 510 may include a user interface for use by the radiologist assisting in performing corresponding selections. If required, the composer component 510 may be usable to adjust acquisition parameters of the selected reference image or to adjust the sequence of reference images of a set (e.g., by adding/removing reference images from the set) to thereby create a patient specific examination. The composer component 510 may be operated by the radiologist at the radiological center or from remote.

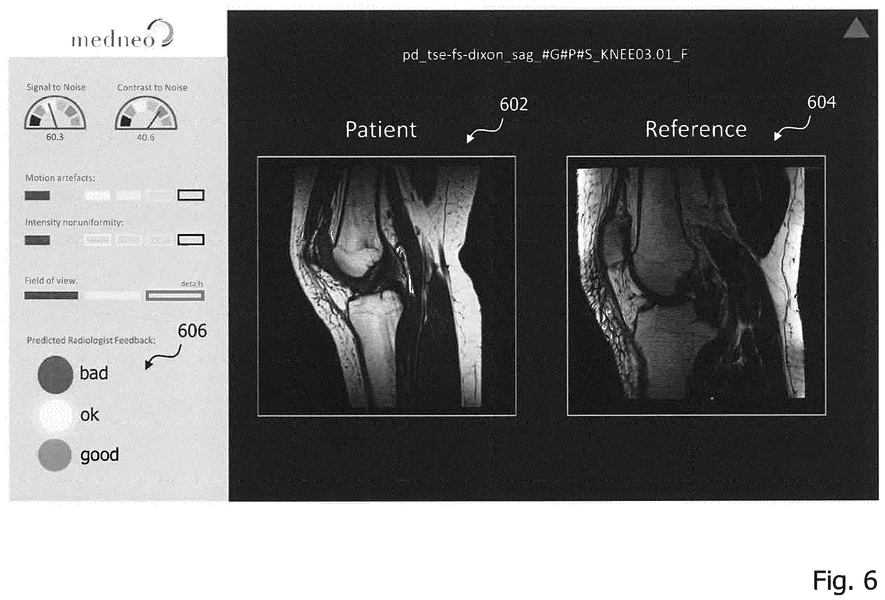

The selected reference image or set may then be sent to the medical imaging device 512, e.g., via a DICOM-based transfer, and the medical imaging device 512 may automatically be configured based on the selected reference image or set. The reference image or set may also be displayed in a DICOM browser at the medical imaging device 512 together with the corresponding SOP. The technician at the medical imaging device 512 may thus prepare the acquisition in accordance with the SOP and perform the actual acquisition process as usual. At the end of the acquisition process, the medical imaging device 512 may send the acquired image or images to the cockpit component 514 (corresponding to the "assessment component" described above) which may determine a corresponding image quality level. The cockpit component 514 may provide a user interface to the technician to display the result of the image quality level assessment, e.g., including an indication of the predicted satisfaction level of the radiologist in charge, so that the technician may know whether or not the radiologist will prospectively be satisfied with the acquired medical image or images. Such display is exemplarily illustrated in Figure 6, where the acquired medical image 602 is displayed next to the corresponding reference image 604 for visual comparison by the technician, and where the predicted satisfaction level of the radiologist is displayed in a traffic light-like manner in the lower left portion of the figure (cf. reference numeral 606). In the upper left portion of the figure, additional quality measurement results are displayed, which may be calculated based on conventional quantitative quality measurements, for example. In the example shown, these measurements comprise assessments of the signal-to-noise ratio, contrast-to-noise ratio, motion artifacts, intensity of nonuniformity, and field of view.

Based on these results, in particular based on the predicted satisfaction level of the radiologist, the technician may decide on whether the acquisition needs to be repeated or whether the image or images may be sent to the radiologist for review. As described above, the predicted satisfaction level of the radiologist may be determined using artificial intelligence-based techniques like deep learning and vector machines, for example. In particular, corresponding algorithms may be trained to predict the satisfaction of a group of radiologists or a single radiologist after an adequate amount of feedback has been collected. The acquired image or images may be sent to the radiologist who may read the image as usual. During the reading process, the radiologist may rate the image quality based on his own assessment on a panel component 516 (corresponding to the "feedback component" described above). The panel component 516 may provide corresponding input means for the radiologist to submit this feedback. The feedback may then be stored (e.g., in the PACS 502 or another dedicated storage), monitored at a dashboard component 518, and used to improve the identification of images with good acquisition parameters for the selection of future reference images as well as to optimize future satisfaction level predictions of radiologists. The feedback given by the radiologists may thus provide a basis for continuous improvements of the reference images and, hence, of the quality level of images acquired in the medical imaging system in general.

As has become apparent from the above, the present disclosure provides a technique for configuring a medical imaging device by a set of acquisition parameters for acquiring a medical image and for performing a feedback-based quality assessment of the acquired medical image. According to the presented technique, acquisition parameters are defined based on reference images rather than based in the conventional generic acquisition protocols locally stored on the modalities. On-site technicians may thus not need to choose a generic acquisition protocol from a locally installed library and mistakes, such as applying wrong acquisition parameters, may thus generally be avoided. By synchronizing reference images from a centrally managed repository across different radiological centers, it may further be ensured that the same acquisition parameters are applied across all sites and that obsolete acquisition parameters are consistently removed from all sites. Patient specific adjustments may be defined by radiologists at their desks and may not have to be applied by the on-site technicians so that inappropriate adjustments of acquisition parameters may eliminated. Besides the reference images, corresponding SOPs associated with the acquisition parameters may be stored in the central repository and presented to the technician prior to image acquisition. Applied SOPs may thus always be up-to-date and assist the technicians in properly performing tasks, such as positioning the patient in the scanner or preparing the acquisition accordingly.

By the technique presented herein, the technician may be presented with predicted satisfaction levels of the radiologists in charge for the acquired images. The predicted satisfaction levels may not only be assessed quantitatively but is based on feedback of radiologists collected for previously acquired medical images, which may reflect the required image quality more precisely and which may even reflect subjective preferences of the radiologists, if desired. Technicians may additionally be notified about image artifacts, wrong fields of view, or other image quality issues and may thus decide on whether the acquisition should be re-run before the images are sent to the radiologist and as long as the patient is still in the scanner. The feedback provided by radiologists may systematically be collected, monitored and incorporated into the selection process of reference images and used to refine the prediction for the satisfaction levels of radiologists. In this way, improvement potential for reference images may be detected in a continuous feedback loop and contribute to improving the overall image quality available in the system.

## Claims

1. A method for performing a feedback-based quality assessment for a medical image acquired by a medical imaging device (512) comprised in a medical imaging system, the method comprising:
selecting (S102), by a user, a reference image (604) from a plurality of reference images (604), each of the plurality of reference images (604) being stored in association with a set of acquisition parameters which has been used to acquire the respective reference image (604), wherein each of the plurality of reference images (604) is previously selected as being representative of a desired level of image quality for a particular type of medical image; **characterized in that** the following steps are carried out:
configuring (S104) the medical imaging device (512) by the set of acquisition parameters stored in association with the selected reference image (604) for acquiring the medical image (602) so that the medical imaging device (512) is configured by a same configuration that was used to acquire the selected reference image (604), wherein the set of acquisition parameters comprises at least one of a repetition time, an echo time, a number of signal averages, a slice thickness, an acquisition plane and a field of view to be applied to the medical imaging device (512) for acquiring the medical image (602);
acquiring (S106) the medical image (602) by the medical imaging device (512), wherein acquiring (S106) the medical image (602) is performed as part of an examination of a patient;
determining (S108) a level of image quality for the acquired medical image (602) using an assessment component (514) comprised in the medical imaging system and configured to assess image quality levels based on feedback of physicians on image quality levels of previously acquired medical images collected for the previously acquired medical images; and
repeating (S112) acquiring the medical image (602) by the medical imaging device (512) with a different configuration within the examination without the patient leaving the medical imaging device (512) when the determined level of image quality does not exceed a predetermined threshold, wherein acquiring the medical image (602) is performed repeatedly until the determined level of image quality exceeds the predetermined threshold, and transmitting (S110) the acquired medical image (602) to a workstation of a physician for review by the physician working from a remote location when the determined level of image quality exceeds the predetermined threshold,
wherein the assessment component (514) comprises a machine learning module trained to predict a satisfaction level of a physician or a group of physicians based on the feedback of physicians on image quality levels of previously acquired medical images collected for the previously acquired medical images, wherein the method comprises collecting (S114) feedback on the image quality level of the acquired medical image (602) from the physician and using the collected feedback for training the machine learning module,
wherein the method further comprises using the collected feedback for selecting reference images (604) representative of desired image quality levels for particular types of medical images.

2. The method of claim 1, wherein the medical imaging device (512) is one of a plurality of medical imaging devices (512) distributed across a plurality of sites and wherein the plurality of reference images (604) is stored in a central repository (504) configured to distribute the plurality of reference images (604) to the plurality of medical imaging devices (512).

3. The method of claim 2, wherein, in each of the plurality of sites, a local subset of the central repository (504) is maintained, wherein each local subset is synchronized with the central repository (504).

4. The method of claim 2 or 3, wherein, in the central repository (504), the plurality of reference images (604) is categorized by at least one of:
medical imaging device types,
standard image sets and/or physician specific image sets,
body regions, and
anatomical parts.

5. The method of any one of claims 1 to 4, wherein each of the plurality of reference images (604) is stored in association with a standard operating procedure, SOP, to be adhered to when acquiring the medical image (602) using the set of acquisition parameters stored in association with the respective reference image (604) .

6. The method of any one of claims 2 to 5, wherein, in the central repository (504), sequences of reference images (604) are defined, wherein the medical imaging device (512) is configurable by each of the sequences to acquire a respective series of medical images (602) in accordance with the sets of acquisition parameters stored in association with the reference images (604) of the respective sequence.

7. The method of claim 6, wherein reference images (604) of the sequences are stored by reference to corresponding reference images (604) of the plurality of reference images (604).

8. A system for performing a feedback-based quality assessment for a medical image, the system comprising:
a composition component comprised in a medical imaging system and configured to obtain a selection (S102) of a reference image (604) selected from a plurality of reference images (604) by a user, each of the plurality of reference images (604) being stored in association with a set of acquisition parameters which has been used to acquire the respective reference image (604), wherein each of the plurality of reference images (604) is previously selected as being representative of a desired level of image quality for a particular type of medical image; **characterized in** the following devices and components:
a configuration component comprised in the medical imaging system and configured to configure (S104) a medical imaging device (512) by the set of acquisition parameters stored in association with the selected reference image (604) for acquiring the medical image (602) so that the medical imaging device (512) is configured by a same configuration that was used to acquire the selected reference image (604), wherein the set of acquisition parameters comprises at least one of a repetition time, an echo time, a number of signal averages, a slice thickness, an acquisition plane and a field of view to be applied to the medical imaging device (512) for acquiring the medical image (602);
the medical imaging device (512) comprised in the medical imaging system and configured to acquire (S106) a medical image (602), wherein acquiring (S106) the medical image (602) is performed as part of an examination of a patient; and
an assessment component (514) comprised in the medical imaging system and configured to determine (S108) a level of image quality for the medical image (602) acquired by the medical imaging device (512), wherein the assessment component (514) is configured to assess image quality levels based on feedback of physicians on image quality levels of previously acquired medical images collected for the previously acquired medical images,
wherein the medical imaging device (512) is configured to repeat (S112) acquiring the medical image (602) by the medical imaging device (512) with a different configuration within the examination without the patient leaving the medical imaging device (512) when the determined level of image quality does not exceed a predetermined threshold, wherein the medical imaging device (512) is configured to perform acquiring the medical image (602) repeatedly until the determined level of image quality exceeds the predetermined threshold, wherein the assessment component (514) is further configured to transmit (S110) the acquired medical image (602) to a workstation of a physician for review by the physician working from a remote location when the determined level of image quality exceeds a predetermined threshold, and
wherein the assessment component (514) comprises a machine learning module trained to predict a satisfaction level of a physician or a group of physicians based on the feedback of physicians on image quality levels of previously acquired medical images collected for the previously acquired medical images, wherein the assessment component (514) is configured to collect (S114) feedback on the image quality level of the acquired medical image (602) from the physician and to use the collected feedback for training the machine learning module,
wherein the assessment component (514) is further configured to use the collected feedback for selecting reference images (604) representative of desired image quality levels for particular types of medical images.

## Patentansprüche

1. Verfahren zur Durchführung einer rückkopplungsbasierten Qualitätsbeurteilung für ein medizinisches Bild, das von einer medizinischen Bildgebungsvorrichtung (512) eines medizinischen Bildgebungssystems erfasst wurde, wobei das Verfahren umfasst:
Auswählen (S102) eines Referenzbildes (604) aus einer Vielzahl von Referenzbildern (604) durch einen Benutzer, wobei jedes der Vielzahl von Referenzbildern (604) in Assoziation zu einem Satz von Erfassungsparametern gespeichert ist, der verwendet wurde, um das jeweilige Referenzbild (604) zu erfassen, wobei jedes der Vielzahl von Referenzbildern (604) zuvor als repräsentativ für ein gewünschtes Bildqualitätsniveau für einen bestimmten Typ eines medizinischen Bildes ausgewählt wird;
**dadurch gekennzeichnet, dass** die folgenden Schritte ausgeführt werden:
Konfigurieren (S104) der medizinischen Bildgebungsvorrichtung (512) durch den Satz von Erfassungsparametern, die in Assoziation zu dem ausgewählten Referenzbild (604) gespeichert sind, um das medizinische Bild (602) zu erfassen, so dass die medizinische Bildgebungsvorrichtung (512) durch eine selbe Konfiguration konfiguriert wird, die verwendet wurde, um das ausgewählte Referenzbild (604) zu erfassen, wobei der Satz von Erfassungsparametern mindestens eines aus einer Wiederholungszeit, einer Echozeit, einer Anzahl von Signalmittelwerten, einer Schichtdicke, einer Erfassungsebene und einem Sichtfeld umfasst, die auf die medizinische Bildgebungsvorrichtung (512) zur Erfassung des medizinischen Bildes (602) anzuwenden sind;
Erfassen (S106) des medizinischen Bildes (602) durch die medizinische Bildgebungsvorrichtung (512), wobei das Erfassen (S106) des medizinischen Bildes (602) als Teil einer Untersuchung eines Patienten durchgeführt wird;
Bestimmen (S108) eines Bildqualitätsniveaus für das erfasste medizinische Bild (602) unter Verwendung einer Bewertungskomponente (514), die in dem medizinischen Bildgebungssystem enthalten ist und konfiguriert ist, um Bildqualitätsniveaus auf der Grundlage von Rückmeldungen von Ärzten über Bildqualitätsniveaus von zuvor erfassten medizinischen Bildern zu bewerten, die für die zuvor erfassten medizinischen Bilder gesammelt wurden; und
Wiederholen (S112) des Erfassens des medizinischen Bildes (602) durch die medizinische Bildgebungsvorrichtung (512) mit einer anderen Konfiguration innerhalb der Untersuchung, ohne dass der Patient die medizinische Bildgebungsvorrichtung (512) verlässt, wenn das ermittelte Bildqualitätsniveau einen vorbestimmten Schwellenwert nicht überschreitet, wobei das Erfassen des medizinischen Bildes (602) wiederholt durchgeführt wird, bis das ermittelte Bildqualitätsniveau den vorbestimmten Schwellenwert überschreitet, und Übertragen (S110) des erfassten medizinischen Bildes (602) an eine Arbeitsstation eines Arztes zur Überprüfung durch den Arzt, der von einem entfernten Ort aus arbeitet, wenn das ermittelte Bildqualitätsniveau den vorbestimmten Schwellenwert überschreitet,
wobei die Bewertungskomponente (514) ein maschinelles Lernmodul umfasst, das so trainiert ist, dass es ein Zufriedenheitsniveau eines Arztes oder einer Gruppe von Ärzten auf der Grundlage der Rückmeldungen von Ärzten zu Bildqualitätsniveaus von zuvor erfassten medizinischen Bildern, die für die zuvor erfassten medizinischen Bilder gesammelt wurden, vorhersagt, wobei das Verfahren ein Sammeln (S114) einer Rückmeldung zu dem Bildqualitätsniveau des erfassten medizinischen Bildes (602) von dem Arzt und ein Verwenden der gesammelten Rückmeldung zum Trainieren des maschinellen Lernmoduls umfasst,
wobei das Verfahren ferner ein Verwenden der gesammelten Rückmeldungen zum Auswählen von Referenzbildern (604) umfasst, die für gewünschte Bildqualitätsniveaus für bestimmte Arten von medizinischen Bildern repräsentativ sind.

2. Verfahren nach Anspruch 1, wobei die medizinische Bildgebungsvorrichtung (512) eine aus mehreren medizinischen Bildgebungsvorrichtungen (512) ist, die über mehrere Standorte verteilt sind, und wobei die Vielzahl von Referenzbildern (604) in einem zentralen Speicher (504) gespeichert sind, der dazu eingerichtet ist, die Vielzahl von Referenzbildern (604) an die mehreren medizinischen Bildgebungsvorrichtungen (512) zu verteilen.

3. Verfahren nach Anspruch 2, wobei an jedem der mehreren Standorte eine lokale Teilmenge des zentralen Speichers (504) verwaltet wird, wobei jede lokale Teilmenge mit dem zentralen Speicher (504) synchronisiert wird.

4. Verfahren nach Anspruch 2 oder 3, wobei in dem zentralen Speicher (504) die Vielzahl von Referenzbildern (604) nach mindestens einem aus dem Folgenden kategorisiert ist:
Typen von medizinischen Bildgebungsgeräten,
Standardbildsätze und/oder arztspezifische Bildsätze,
Körperregionen, und
anatomischen Teile.

5. Verfahren nach einem der Ansprüche 1 bis 4, wobei jedes der Vielzahl von Referenzbildern (604) in Verbindung mit einer Standardarbeitsanweisung, SOP, gespeichert ist, die bei der Erfassung des medizinischen Bildes (602) unter Verwendung des Satzes von Erfassungsparametern, die in Assoziation zu dem jeweiligen Referenzbild (604) gespeichert sind, einzuhalten ist.

6. Verfahren nach einem der Ansprüche 2 bis 5, wobei in dem zentralen Speicher (504) Sequenzen von Referenzbildern (604) definiert sind, wobei die medizinische Bildgebungsvorrichtung (512) durch jede der Sequenzen konfigurierbar ist, um eine jeweilige Serie von medizinischen Bildern (602) in Übereinstimmung mit den Sätzen von Aufnahmeparametern, die in Verbindung mit den Referenzbildern (604) der jeweiligen Sequenz gespeichert sind, aufzunehmen.

7. Verfahren nach Anspruch 6, wobei Referenzbilder (604) der Sequenzen durch Verweis auf entsprechende Referenzbilder (604) der Vielzahl von Referenzbildern (604) gespeichert sind.

8. System zur Durchführung einer rückkopplungsbasierten Qualitätsbeurteilung für ein medizinisches Bild, wobei das System umfasst:
eine Kompositionskomponente, die in einem medizinischen Bildgebungssystem enthalten ist und so konfiguriert ist, dass sie eine Auswahl (S102) eines Referenzbildes (604) erhält, das durch einen Benutzer aus einer Vielzahl von Referenzbildern (604) ausgewählt wird, wobei jedes der Vielzahl von Referenzbildern (604) in Assoziation zu einem Satz von Erfassungsparametern gespeichert ist, der verwendet wurde, um das jeweilige Referenzbild (604) zu erfassen, wobei jedes der Vielzahl von Referenzbildern (604) zuvor als repräsentativ für ein gewünschtes Bildqualitätsniveau für einen bestimmten Typ eines medizinischen Bildes ausgewählt wird;
**gekennzeichnet durch** die folgenden Vorrichtungen und Komponenten:
eine Konfigurationskomponente, die in dem medizinischen Bildgebungssystem enthalten ist und so konfiguriert ist, dass sie eine medizinische Bildgebungsvorrichtung (512) durch den Satz von Erfassungsparametern konfiguriert (S104), die in Assoziation zu dem ausgewählten Referenzbild (604) gespeichert sind, um das medizinische Bild (602) zu erfassen, so dass die medizinische Bildgebungsvorrichtung (512) durch eine selbe Konfiguration konfiguriert wird, die verwendet wurde, um das ausgewählte Referenzbild (604) zu erfassen, wobei der Satz von Erfassungsparametern mindestens eines aus einer Wiederholungszeit, einer Echozeit, einer Anzahl von Signalmittelwerten, einer Schichtdicke, einer Erfassungsebene und einem Sichtfeld umfasst, die auf die medizinische Bildgebungsvorrichtung (512) zur Erfassung des medizinischen Bildes (602) anzuwenden sind;
die medizinische Bildgebungsvorrichtung (512), die in dem medizinischen Bildgebungssystem enthalten ist und so konfiguriert ist, dass sie ein medizinisches Bild (602) erfasst (S106), wobei das Erfassen (S106) des medizinischen Bildes (602) als Teil einer Untersuchung eines Patienten durchgeführt wird;
eine Bewertungskomponente (514), die in dem medizinischen Bildgebungssystem enthalten ist und so konfiguriert ist, dass sie ein Bildqualitätsniveau für das erfasste medizinische Bild (602) bestimmt (S108), wobei die Bewertungskomponente (514) so konfiguriert ist, dass sie Bildqualitätsniveaus auf der Grundlage von Rückmeldungen von Ärzten über Bildqualitätsniveaus von zuvor erfassten medizinischen Bildern bewertet, die für die zuvor erfassten medizinischen Bilder gesammelt wurden;
wobei die medizinische Bildgebungsvorrichtung (512) so konfiguriert ist, dass sie das Erfassens des medizinischen Bildes (602) mit einer anderen Konfiguration innerhalb der Untersuchung wiederholt (S112), ohne dass der Patient die medizinische Bildgebungsvorrichtung (512) verlässt, wenn das ermittelte Bildqualitätsniveau einen vorbestimmten Schwellenwert nicht überschreitet, wobei die medizinische Bildgebungsvorrichtung (512) so konfiguriert ist, dass sie das Erfassen des medizinischen Bildes (602) wiederholt durchführt, bis das ermittelte Bildqualitätsniveau den vorbestimmten Schwellenwert überschreitet, wobei die Bewertungskomponente (514) ferner so konfiguriert ist, dass sie das erfasste medizinische Bild (602) an eine Arbeitsstation eines Arztes zur Überprüfung durch den Arzt überträgt (S110), der von einem entfernten Ort aus arbeitet, wenn das ermittelte Bildqualitätsniveau den vorbestimmten Schwellenwert überschreitet, und
wobei die Bewertungskomponente (514) ein maschinelles Lernmodul umfasst, das so trainiert ist, dass es ein Zufriedenheitsniveau eines Arztes oder einer Gruppe von Ärzten auf der Grundlage der Rückmeldungen von Ärzten zu Bildqualitätsniveaus von zuvor erfassten medizinischen Bildern, die für die zuvor erfassten medizinischen Bilder gesammelt wurden, vorhersagt, wobei die Bewertungskomponente (514) so konfiguriert ist, dass sie eine Rückmeldung zu dem Bildqualitätsniveau des erfassten medizinischen Bildes (602) von dem Arzt sammelt (S114) und die gesammelte Rückmeldung zum Trainieren des maschinellen Lernmoduls verwendet,
wobei die Bewertungskomponente (514) ferner so konfiguriert ist, dass sie die gesammelten Rückmeldungen zum Auswählen von Referenzbildern (604) verwendet, die für gewünschte Bildqualitätsniveaus für bestimmte Arten von medizinischen Bildern repräsentativ sind.

## Revendications

1. Procédé d'évaluation de la qualité fondée sur un retour d'information concernant une image médicale acquise par un dispositif d'imagerie médicale (512) compris dans un système d'imagerie médicale, le procédé consistant à :
sélectionner (S102), par un utilisateur, une image de référence (604) parmi une pluralité d'images de référence (604), chaque image de référence de la pluralité des images de référence (604) étant stockée en association avec un ensemble de paramètres d'acquisition qui a été utilisé pour acquérir l'image de référence respective (604), dans lequel chaque image de référence de la pluralité des images de référence (604) est sélectionnée préalablement comme représentant un niveau souhaité de la qualité d'image pour un type particulier d'image médicale ;
**caractérisé en ce que** les étapes suivantes étant réalisées :
la configuration (S104) du dispositif d'imagerie médicale (512) par l'ensemble des paramètres d'acquisition stocké en association avec l'image de référence sélectionnée (604) pour acquérir l'image médicale (602) de manière à ce que le dispositif d'imagerie médicale (604) soit configurée par une même configuration qui a été utilisée pour acquérir l'image de référence sélectionnée (604), dans lequel l'ensemble des paramètres d'acquisition comprend au moins l'un parmi un temps de répétition, un temps d'écho, un certain nombre de moyennes de signal une épaisseur de tranche, un plan d'acquisition et un champ de vision à appliquer au dispositif d'imagerie médicale (512) pour acquérir l'image médicale (602) ;
l'acquisition (S106) de l'image médicale (602) par le dispositif d'imagerie médicale (512), l'acquisition (S106) de l'image médicale (602) est effectuée comme faisant partie d'un examen d'un patient ;
la détermination (S108) d'un niveau de qualité d'image pour l'image médicale acquise (602) à l'aide d'un composant d'évaluation (514) compris dans le système d'imagerie médicale et configurée pour évaluer les niveaux de qualité d'image en fonction d'un retour d'information provenant des médecins concernant les niveaux de qualité d'image des images médicales acquises préalablement collectées pour les images médicales acquises précédemment ; et
la répétition (S112) de l'acquisition de l'image médicale (602) par le dispositif d'imagerie médicale (512) au moyen d'une configuration différente dans le cadre de l'examen sans que le patient ne quitte le dispositif d'imagerie médicale (512) lorsque le niveau déterminé de qualité d'image ne dépasse pas un seuil prédéfini, dans lequel l'acquisition de l'image médicale (602) est effectuée de manière répétée jusqu'à ce que le niveau de qualité d'image prédéfini dépasse le seuil prédéfini, et la transmission (S110) de l'image médicale acquise (602) à un poste de travail d'un médecin pour révision par le médecin travaillant depuis un lieu à distance lorsque le niveau de qualité d'image déterminé dépasse le seuil prédéfini,
dans lequel le composant d'évaluation (514) comprend un module d'apprentissage automatique entraîné pour prédire un niveau de satisfaction d'un médecin ou d'un groupe de médecins en fonction du retour d'information de médecins concernant les niveaux de qualité d'image des images médicales acquises précédemment collectées pour les images médicales acquises précédemment, dans lequel le procédé consiste à collecter (S114) des informations en retour concernant le niveau de qualité d'image de l'image médicale acquise (602) provenant du médecin et à utiliser le retour d'information collecté pour entraîner le module d'apprentissage automatique,
dans lequel le procédé consiste en outre à utiliser le retour d'information collecté pour sélectionner des images de référence (604) représentatives des niveaux de qualité d'image souhaités pour des types particuliers d'images médicales.

2. Procédé selon la revendication 1, dans lequel le dispositif d'imagerie médicale (512) est un dispositif parmi la pluralité de dispositifs d'imagerie médicale (512) distribués dans l'ensemble d'une pluralité d'emplacements et dans lequel la pluralité des images de références (604) est stockée dans un référentiel de données central (504) configuré pour distribuer la pluralité des images de référence (604) à la pluralité des dispositifs d'imagerie médicale (512).

3. Procédé selon la revendication 2, dans lequel dans chaque emplacement de la pluralité des emplacements, un sous-ensemble local du référentiel de données central (504) est conservé, chaque sous-ensemble local étant synchronisé avec le référentiel de données central (504).

4. Procédé selon la revendication 2 ou 3, dans lequel, dans le référentiel de données central (504), la pluralité des images de référence (604) est catégorisée par l'au moins un élément parmi :
des types de dispositif d'imagerie médicale,
des ensembles d'images standard et/ou des ensembles d'images spécifiques de médecin,
des parties de corps, et
des parties anatomiques.

5. Procédé selon l'une quelconque des revendication 1 à 4, dans lequel chaque image de référence de la pluralité des images de référence (604) est stockée en association avec une procédure de fonctionnement standard, SOP, qui doit être observée au moment de l'acquisition de l'image médicale (602) à l'aide de l'ensemble des paramètres d'acquisition stockés en association avec l'image de référence respective (604).

6. Procédé selon l'une quelconque des revendication 2 à 5, dans lequel, dans le référentiel de données central (504), sont définies des séquences d'images de référence (604), dans lequel le dispositif d'imagerie médicale (512) peut être configuré par chacune des séquences pour acquérir une série respective d'images médicales (602) conformément aux ensembles de paramètres d'acquisition stockés en association des images de référence (604) de la séquence respective.

7. Procédé selon la revendication 6, dans lequel les images de référence (604) des séquences sont stockées en référence aux images de référence correspondantes (604) de la pluralité des images de référence (604).

8. Système pour effectuer l'évaluation de qualité fondée sur un retour d'information pour une image médicale, le système comprenant :
un composant de composition compris dans un système d'imagerie médicale et configuré pour obtenir une sélection (S102) d'une image de référence (604) sélectionnée parmi une pluralité d'images de référence (604) par un utilisateur, chaque image de référence de la pluralité d'images de référence (604) étant stockée en association avec un ensemble de paramètres d'acquisition qui ont été utilisés pour acquérir l'image de référence respective (604), dans lequel chaque image de référence de la pluralité des images de référence (604) est sélectionnée précédemment comme représentant un niveau de qualité d'image souhaité pour un type particulier d'image médicale ;
**caractérisé par** les dispositifs et composants suivants :
un composant de configuration compris dans le système d'imagerie médicale et configuré pour configurer (S104) un dispositif d'imagerie médicale (512) par l'ensemble des paramètres d'acquisition stocké en association avec l'image de référence sélectionnée (604) pour acquérir l'image médicale (602) de manière à ce que le dispositif d'imagerie médicale (512) soit configuré par une même configuration qui a été utilisée pour acquérir l'image de référence sélectionnée (604), dans lequel l'ensemble des paramètres d'acquisition comprend au moins l'un parmi un temps de répétition, un temps d'écho, un certain nombre de moyennes de signal une épaisseur de tranche, un plan d'acquisition et un champ de vision à appliquer au dispositif d'imagerie médicale (512) pour acquérir l'image médicale (602) ;
le dispositif d'imagerie médicale (512) compris dans le système d'imagerie médicale et configuré pour acquérir une image médicale (602), dans lequel l'acquisition (S106) de l'image médicale (602) est effectuée comme faisant partie d'un examen d'un patient ; et
un composant d'évaluation (514) compris dans le système d'imagerie médicale et configuré pour déterminer (S108) un niveau de qualité d'image pour l'image médicale acquise (602) à l'aide d'un composant d'évaluation (514) compris dans le système d'imagerie médicale et configuré pour évaluer les niveaux de qualité d'image en fonction d'un retour d'information provenant des médecins concernant les niveaux de qualité d'image des images médicales acquises préalablement collectées pour les images médicales acquises précédemment ;
dans lequel le dispositif d'imagerie médicale (512) est configuré pour répéter (S112) l'acquisition de l'image médicale (602) par le dispositif d'imagerie médicale (512) à l'aide d'une configuration différente à l'intérieur de l'examen sans que le patient ne quitte le dispositif d'imagerie médicale (512) lorsque le niveau déterminé de qualité d'image ne dépasse pas un seuil prédéfini, dans lequel l'acquisition de l'image médicale (602) est effectuée de manière répétée jusqu'à ce que le niveau de qualité d'image prédéfini dépasse le seuil prédéfini, et la transmission (S110) de l'image médicale acquise (602) à un poste de travail d'un médecin pour révision par le médecin travaillant depuis un lieu à distance lorsque le niveau de qualité d'image déterminé dépasse le seuil prédéfini, et
dans lequel le composant d'évaluation (514) comprend un module d'apprentissage automatique entraîné pour prédire un niveau de satisfaction d'un médecin ou d'un groupe de médecins en fonction du retour d'information de médecins concernant les niveaux de qualité d'image des images médicales acquises précédemment collectées pour les images médicales acquises précédemment, dans lequel le procédé consiste à collecter (S114) des informations en retour concernant le niveau de qualité d'image de l'image médicale acquise (602) provenant du médecin et à utiliser les informations en retour collectées pour entraîner le module d'apprentissage automatique,
dans lequel le composant d'évaluation (514) est en outre configuré pour utiliser les informations en retour collectées pour sélectionner des images de référence (604) représentatives des niveaux de qualité d'image souhaités pour des types particuliers d'images médicales.
